# EUROPÄISCHE PATENTSCHRIFT

(11) **EP 3 274 465 B1**
(45) Veröffentlichungstag und Bekanntmachung des Hinweises auf die Patenterteilung: **03.08.2022**
(21) Anmeldenummer: 16705208.3
(22) Anmeldetag: 19.02.2016
(51) Int. Cl.: C12P 19/02, C12N 9/04, C12N 9/08

(54) **BIOKATALYTISCHE HERSTELLUNG VON L-FUCOSE**
BIOCATALYTIC PREPARATION OF L-FUCOSE
PRODUCTION BIO-CATALYTIQUE DE FUCOSE L

(30) Priorität: 26.03.2015 EP 15160945
(43) Veröffentlichungstag der Anmeldung: 31.01.2018
(73) Patentinhaber: BASF SE, 67056 Ludwigshafen am Rhein (DE)
(72) Erfinder: NAVICKAS, Vaidotas, 68199 Mannheim (DE); BREUER, Michael, 64285 Darmstadt (DE); PUHL, Michael, 69493 Hirschberg (DE); WEINGARTEN, Melanie, 23909 Ratzeburg (DE); BALDENIUS, Kai-Uwe, 68159 Mannheim (DE); SIEGEL, Wolfgang, 67117 Limburgerhof (DE)
(86) Internationale Anmeldenummer: PCT/EP2016/053591
(87) Internationale Veröffentlichungsnummer: WO 2016/150629

(56) Entgegenhaltungen:
- WO-A1-2010/022244
- US-B2- 7 575 910
- US-B2- 8 642 297
- PARIKKA K ET AL: "Oxidation of methyl alpha-d-galactopyranoside by galactose oxidase: products formed and optimization of reaction conditions for production of aldehyde", CARBOHYDRATE RESEARCH, PERGAMON, GB, Bd. 344, Nr. 1, 5. Januar 2009 (2009-01-05), Seiten 14-20, XP027553397, ISSN: 0008-6215 [gefunden am 2008-08-24]
- KIRSTI PARIKKA ET AL: "Oxidation of Polysaccharides by Galactose Oxidase", JOURNAL OF AGRICULTURAL AND FOOD CHEMISTRY, Bd. 58, Nr. 1, 13. Januar 2010 (2010-01-13), Seiten 262-271, XP055210540, ISSN: 0021-8561, DOI: 10.1021/jf902930t
- ROBERT L ROOT ET AL: "Enzymatic Synthesis of Unusual Sugars: Galactose Oxidase Catalyzed Stereospecific Oxidation of Polyols", JOURNAL OF THE AMERICAN CHEMICAL SOCIETYCIETY|, vol. 107, no. 10, 1985, pages 2997-2999, XP001364548, ISSN: 0002-7863

## Beschreibung

### Technisches Gebiet

Die vorliegende Erfindung betrifft Verfahren für die biokatalytische, d.h. enzymatische und/oder fermentative, Herstellung von L-Fucose aus L-Fucitol. Weiterhin offenbart die vorliegende Erfindung Enzyme, insbesondere eine Galaktose-Oxidase, welche für die biokatalytische Herstellung von L-Fucose aus L-Fucitol geeignet sind. Ferner werden rekombinante Mikroorganismen und Pilze offenbart, welche die für die Enzyme zur Herstellung von L-Fucose aus L-Fucitol benötigten Nukleinsäuresequenzen exprimierbar als Transgen tragen und dieses funktional exprimieren können, sowie die Verwendung dieser Mikroorganismen oder Pilze in den Verfahren gemäß der vorliegenden Erfindung.

### Hintergrund der Erfindung

Fucose kommt in zwei enantiomeren Formen vor: L-Fucose (auch Isodulcit genannt) und D-Fucose (CAS 3615-37-0). Die L-Form ist die in der Natur verbreitete. L-Fucose ist ein elementares Molekül für den Einsatz in Forschung und Entwicklung und eine charakteristische Komponente von Glycan-Strukturen. Wissenschaftliche Untersuchungen im Bereich von fucosylierten Substanzen haben unter anderem gezeigt, dass diese eine herausragende Bedeutung in der embryonalen Entwicklung haben und an der Immunantwort des Organismus beteiligt sind.

L-Fucose ist unter anderem in der menschlichen Muttermilch zu finden. Menschlicher Muttermilch wird eine wichtige Rolle bei der gesunden Kindesentwicklung beigegemessen. Die darin vorkommenden Substanzen, insbesondere die Oligosaccharide (human milk oligosaccharides (HMO)) sind eine der festen Hauptkomponenten der Muttermilch und haben eine Kernstruktur, die eine Lactoseeinheit am reduzierenden Ende aufweist und mit N-Acetyllactosamineinheiten verzweigt oder kettenförmig fortgesetzt wird. Die strukturelle Variabilität wird u.a. durch Fucosyl-Modifikationen an den Endpositionen erweitert. Im Hinblick auf die gesundheits- und entwicklungsfördernde Wirkung ist die biologische Funktion der HMOs Gegenstand zahlreicher Studien, erfordert jedoch die technische Reingewinnung der Verbindungen in ausreichenden Mengen. Derzeit erfolgt die Bereitstellung von L-Fucose entweder durch die direkte Extraktion aus natürlichen Quellen oder durch chemische Modifikation von Monosacchariden (für eine Zusammenfassung, siehe P. T. Vanhooren et al. J. Chem. Technol. Biotechnol. 74, 479 (1999) sowie darin zitiere Quellen). Die aufwendige Extraktion aus Muttermilch ist derzeit die am meisten angewandte Methode. Biotechnologische Produktionsverfahren wurden zwar für HMOs beschrieben (siehe Han et al., Biotechnol. Adv. 2012, 30, 1268-1278), jedoch sind die entsprechenden HMOs häufig nicht zu einem angemessenen Preis erhältlich.

L-Fucose sowie L-Fucose enthaltende Substanzen sind Gegenstand laufender Forschungsarbeiten. L-Fucose selbst sowie fucosylierte Substrate sind wichtige Ausgangsstoffe in der chemischen und pharmazeutischen Industrie sowie nützlich bei der Herstellung von Kosmetika und Nutrazeutika. Daher besteht ein ständiger Bedarf an innovativen Produktionsprozessen für die Herstellung von L-Fucose im industriellen Maßstab.

Es wurden biotechnologische Verfahren vorgeschlagen, um L-Fucose zu gewinnen. So lehrt etwa WO 2012/034996 A1 ein Verfahren zur Herstellung von L-Fucose unter Verwendung eines isolierten Mikroorganismus der Familie Enterobacteriaceae (hinterlegt als DSM 22227), welcher über eine spezielle 16S rRNA gemäß SEQ ID NO:1 verfügt, zur fermentativen Herstellung von L-Fucose. Die L-Fucose wird jedoch erst nach zeitaufwendiger Fermentation als nicht gereinigte Mischung erhalten, weshalb aufwendige weitere Reinigungsschritte erforderlich sind, um L-Fucose zu erhalten. US 8,642,297 B2 postuliert *inter alia* ein generisches fermentatives Verfahren, welches zur Herstellung von L-Fucose unter Verwendung einer rekombinanten Mannitol-1-Dehydrogenase aus *Apium graveolens* führen soll. Hierzu wird jedoch weder ein konkretes Ausführungsbeispiel noch ein Beispiel offenbart. Ferner wird kein alternatives Enzym offenbart, welches für die Reaktion eingesetzt werden könnte. WO 2005/087941 A1 offenbart ein kombiniertes fermentativ-enzymatisches Verfahren zur Bereitstellung von L-Fucose. Hierbei wird zunächst durch Einsatz einer Dehydrogenase eines Acetobakteriums L-Fuculose aus L-Fucitol erzeugt. Diese muss dann in einem weiteren Schritt zu L-Fucose synthetisiert werden. WO 2010/022244 A1 offenbart ein Verfahren zur Herstellung von L-Fucitol mittels Mikroorganismen, die eine Polyol-1-dehydrogenase exprimieren.

R. Root et al. (J. Am. Chem. Soc. 1985, 107, 10, 2997-2999) offenbart die Herstellung ungewöhnlicher Zucker unter Verwendung einer Galaktose-Oxidase, bezeichnet allerdings das dem Fucitol sehr ähnliche Galactitol als schlechtes Substrat in den offenbarten Verfahren.

Es besteht daher nach wie vor ein großes Bedürfnis danach, L-Fucose in ausreichender Reinheit in einem großtechnisch einsetzbaren Verfahren wirtschaftlich in möglichst wenigen Schritten produzieren zu können.

### Zusammenfassung der Erfindung

Der vorliegenden Erfindung liegt daher die Aufgabe zu Grunde, ein Verfahren zur biotechnologischen Herstellung von L-Fucose mit hierfür geeigneten Enzymen und rekombinante Mikroorganismen und Pilze bereitzustellen, wobei L-Fucose unmittelbar in einer Ein-Schritt-Reaktion aus L-Fucitol im industriellen Maßstab gewonnen werden kann.

Die folgende Erfindung löst diese Aufgabe durch die Bereitstellung eines Verfahrens unter Verwendung von L-Fucitol sowie Galaktose-Oxidase sowie einer Peroxidase und einer Katalase, wodurch die Umsetzung von L-Fucitol zu L-Fucose in einer Ein-Schritt-Reaktion durchgeführt und hierbei hohe Ausbeuten erzielt werden können. Weiter offenbart sind hierfür geeignete Enzyme. Schließlich wird ein rekombinanter Mikroorganismus oder Pilz offenbart, welcher die für den offenbarten Syntheseweg relevanten Enzyme produzieren kann sowie dessen Verwendung.

Aspekte und Ausführungsformen der vorliegenden Erfindung ergeben sich aus der folgenden detaillierten Beschreibung und den Beispielen, den Figuren, sowie den beigefügten Patentansprüchen.

### Kurze Beschreibung der Zeichnungen

Figur 1 (Fig. 1) zeigt den Reaktionsweg von Galaktose über L-Fucitol zu L-Fucose, wobei der Schritt der Oxidation des L-Fucitol durch eine Galaktose-Oxidase bewirkt wird.
Figur 2 (Fig. 2) zeigt den Einfluss der Substratkonzentration auf die Umsetzung von L-Fucitol zu L-Fucose unter Verwendung einer Galaktose-Oxidase über die Zeit.
Figur 3 (Fig. 3) zeigt den Einfluss von H₂O₂ auf die Umsetzung von L-Fucitol zu L-Fucose unter Verwendung einer Galaktose-Oxidase über die Zeit.
Figur 4 (Fig. 4) zeigt den Einfluss von L-Fucose auf die Umsetzung von L-Fucitol zu L-Fucose unter Verwendung einer Galaktose-Oxidase über die Zeit.

### Ausführliche Beschreibung

### Definitionen

Die Begriffe Protein, Polypeptid und Enzym werden auf Grund der stets enzymatischen Funktion der hierin offenbarten Polypeptide austauschbar verwendet.

Die Begriffe biokatalytisch oder Biokatalyse, wie hierin verwendet, beziehen sich auf eine Umsetzung und Beschleunigung oder Steuerung von chemischen Reaktionen, in der Enzyme als biologische Katalysatoren dienen. Die Biokatalyse kann entweder durch Zugabe isolierter Enzyme (enzymatisch) oder direkt in lebenden Zellen (hierin: fermentativ) erfolgen.

Der Begriff rekombinanter Mikroorganismus oder Pilz, wie hierin verwendet, bezieht sich auf einen Mikroorganismus oder Pilz, welcher ein rekombinantes, d.h. (teil-)artifizielles Biomolekül von Interesse umfasst, wobei das Biomolekül eine Nukleinsäure- oder eine Polypeptidsequenz sein kann. Ferner kann der gewählte Mikroorganismus- oder Pilz-Stamm Mutationen in seinem Genom oder auf umfasster Plasmid-DNA im Vergleich zu einem nicht veränderten oder zu einem Wildtyp-Stamm tragen, welche nicht die Nukleinsäuresequenz des Biomoleküls von Interesse betreffen.

Der Begriff Transgen wie hierin verwendet bezeichnet ein Nukleinsäuremolekül einer Spezies, welches entweder direkt in das Genom einer anderen Spezies oder mittels eines Vektors außerhalb des Genoms, jedoch replizierbar in die anderen Spezies eingebracht wurde. Der so modifizierte Organismus wird hierein als rekombinant bezeichnet (s.o.).

Der Begriff Abkömmling wie hierin verwendet bezeichnet im Kontext eines rekombinanten Mikroorganismus oder Pilzes gemäß der vorliegenden Offenbarung die Nachkommen eines solchen Organismus, welche durch natürliche reproduktive Vermehrungsvorgänge, umfassend geschlechtliche und ungeschlechtliche, aus dem Ursprungsorganismus hervorgehen. Es ist dem Fachmann auf dem Gebiet bekannt, dass im Zuge der Fortpflanzung auf natürliche Weise Mutationen ins Genom eines Organismus eingeführt werden können, wodurch sich der Abkömmling genomisch vom Elternorganismus unterscheidet, jedoch nach wie vor der gleichen (Sub-)Spezies zugeordnet werden kann. Auch derartige durch natürliche Vorgänge veränderte Abkömmlinge sind daher vom Begriff Abkömmling gemäß der vorliegenden Offenbarung umfasst.

Der Begriff Vektor wie hierin verwendet bezeichnet ein Transportvehikel zur Übertragung eines Nukleinsäuremoleküls in eine Zelle, einen Mikroorganismus oder Pilz. Vektoren können unter anderen Plasmide, Cosmide oder modifizierte Viren sein. Ferner soll der Begriff Vektor auch Mittel umfassen, welcher zur direkten Einführung eines Polypeptids in eine Zelle oder einen Mikroorganismus geeignet sind.

Der Begriff "in funktionaler Form exprimieren" wie hierin verwendet, bezeichnet die Fähigkeit eines rekombinanten Mikroorganismus oder Pilzes, ein oder mehrere Transgen(e), ausgewählt aus der Gruppe, bestehend aus einer Galaktose-Oxidase, einer Katalase und einer Peroxidase, wie oben definiert, translatieren zu können, so dass ein Polypeptid entsteht, welches unter geeigneten Reaktionsbedingungen die enzymatische Funktion einer Galaktose-Oxidase, einer Katalase oder einer Peroxidase erfüllt, wie oben dargelegt.

### Detaillierte Beschreibung der Erfindung

Die vorliegende Erfindung wird mit dem Gegenstand des beigefügten Anspruchssatzes definiert.

In einem Aspekt stellt die vorliegende Erfindung ein Verfahren zur Herstellung von L-Fucose bereit, umfassend folgende Schritte: (a) Bereitstellen von L-Fucitol, (b) Bereitstellen einer Galaktose-Oxidase der Enzymklasse EC 1.1.3.9, einer Peroxidase und einer Katalase, (c) Oxidation von L-Fucitol unter Verwendung einer Galaktose-Oxidase durch Inkubieren der erhaltenen Mischung unter Bedingungen, die die biokatalytische Oxidation von L-Fucitol zu L-Fucose erlauben, (d) optional: Isolieren der synthetisierten L-Fucose.

Die Galaktose-Oxidase (EC 1.1.3.9) ist eine Kupfer-abhängige Oxidase. Sie kommt in der Natur oft als extrazelluläres, monomeres Enzym vor, das von vielen filamentösen Pilzen sekretiert wird. Galaktose-Oxidasen katalysieren die Oxidation primärer Alkohole zu den entsprechenden Aldehyden, gleichzeitig wird molekularer Sauerstoff zu H₂O₂ reduziert (siehe Paukner, R. et al.; Galactose oxidase from Fusarium oxysporum - expression in E. coli and P. pastoris and biochemical characterization; PLoS ONE, 2014, 9, e100116/1). Die Aktivität einer Galaktose-Oxidase lässt sich mit dem Fachmann bekannten Methoden bestimmen, wobei die Freisetzung von Wasserstoffperoxid (H₂O₂) aus Reduktion von Sauerstoff (O₂) in Gegenwart eines geeigneten Substrates (insb. Galaktose) quantifiziert wird. Alternativ ist auch die Verminderung der O₂-Konzentration ein Maß für die Aktivität einer Galaktose-Oxidase.

In einer Ausführungsform umfasst das Verfahren die Teilschritte (b1) Bereitstellen eines rekombinanten Mikroorganismus oder Pilzes zur rekombinanten Expression einer Galaktose-Oxidase der Enzymklasse EC 1.1.3.9, wobei der Mikroorganismus oder Pilz ein Transgen, das für eine Galaktose-Oxidase aus einem Pilz der Ordnung der *Hypocreales,* vorzugsweise ausgewählt aus der Gattung *Fusarium,* insbesondere der Species *Fusarium oxysporum,* kodiert, enthält, (b2) Kultivieren des rekombinanten Mikroorganismus unter Bedingungen, die die Synthese der Galaktose-Oxidase erlauben, (b3) optional: Isolieren der synthetisierten Galaktose-Oxidase. Die gemäß der vorliegenden Offenbarung bevorzugte Galaktose-Oxidase aus *Fusarium oxysporum* ist ein Polypeptid, umfassend 681 Aminosäuren (GenBank^{®} Hinterlegungsnummer: AHA90705.1). Die korrespondierende Nukleinsäuresequenz (2046 Nukleotide inkl. Stop-Codon) der kodierenden Sequenz ist bei der GenBank^{®} als Hinterlegungsnummer KF601698.1 zugänglich.

Es ist dem Fachmann auf dem Gebiet bekannt, dass jedes Enzym der Enzymklasse 1.1.3.9, welche durch eine vergleichbare katalytische Leistung definiert ist, in dem Verfahren gemäß der vorliegenden Erfindung verwendet werden kann, solange das Enzym die oben skizzierte Funktion der Oxidation primärer Alkohole zu den entsprechenden Aldehyden bei gleichzeitiger Reduktion von molekularem Sauerstoff zu H₂O₂ katalysiert. Ebenso ist dem Fachmann auf dem Gebiet bekannt, dass die Reaktionsbedingungen je nach verwendetem Enzym variieren können. Anpassungen der Reaktions- und/oder Kulturbedingungen können vom Fachmann auf dem Gebiet ohne Weiteres vorgenommen werden.

Gemäß der vorliegenden Erfindung wird die biokatalytische Gewinnung von L-Fucose ausgehend von L-Fucitol durch den Einsatz einer Galaktose-Oxidase, einer Peroxidase und eine Katalase gelehrt. Diese biokatalytischen Umsetzungen gemäß der vorliegenden Erfindung kann entweder vollständig enzymatisch, enzymatisch und fermentativ, oder ausschließlich fermentativ erfolgen. Enzymatisch in diesem Zusammenhang bedeutet die Verwendung isolierter und gegebenenfalls aufgereinigter und weiter modifizierter Enzyme. Fermentativ in diesem Kontext bedeutet die Expression von einem oder von mehreren Zielenzym(en) in einem rekombinanten Wirtsorganismus unter geeigneten Reaktionsbedingungen, welche die Expression des Enzyms in funktionsfähiger Form gestatten.

Auf Grund der Tatsache, dass die Reaktion der Umsetzung von L-Fucitol zu L-Fucose unter Einsatz einer Galaktose-Oxidase eine Sauerstoff-abhängige Reaktion ist, ist vorgesehen, dass die Reaktanten stets in ausreichenden Kontakt mit Sauerstoff kommen. Dieser Sauerstoffeintrag kann durch Mittel bewirkt werden, die dem Fachmann auf dem Gebiet wohlbekannt sind, wie zum Beispiel Aeration durch Begasen oder Belüften eines Kultur- oder Reaktionsansatzes.

Katalasen katalysieren die Reaktion der Summengleichung: 2 H₂O₂ → O₂ + 2 H₂O und setzen somit das bei der Reaktion der Galaktose-Oxidase mit L-Fucitol entstehende Nebenprodukt H₂O₂ um. Peroxidasen, wie zum Beispiel Meerrettich-Peroxidase, sind Enzyme, welche die Reduktion von Peroxiden katalysieren. Die Summengleichung der katalysierten Reaktion lautet ebenfalls: 2 H₂O₂ → O₂ + 2 H₂O. Die Substratspezifität von Peroxidasen kann stark variieren. Meerrettich-Peroxidase ist ein beispielhafter Vertreter dieser Enzymklasse mit einem breiten Spektrum an Donatoren und Akzeptoren. Für die Anwendung in der vorliegenden Erfindung ist jede Katalase bzw. Peroxidase geeignet, welche unter geeigneten Reaktionsbedingungen den Abbau von H₂O₂ zu Sauerstoff und Wasser katalysieren kann.

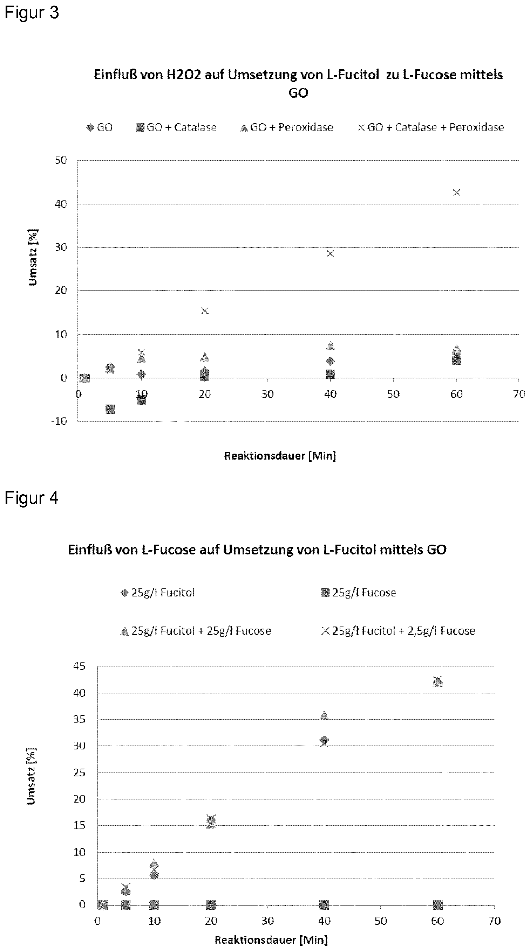

Es wurde herausgefunden, dass die Zugabe einer Peroxidase und/oder einer Katalase die Umsetzung von L-Fucitol zu L-Fucose vorteilhaft beeinflusst, da das unter anderem durch die Aktivität der Galaktose-Oxidase entstehende H₂O₂ durch die Aktivität dieser zusätzlichen H₂O₂ detoxifizierenden Enzyme entfernt und somit die Ausbeute an L-Fucose erhöht werden kann. In allen Ausführungsformen wird sowohl eine Katalase als auch eine Peroxidase zusätzlich zur Galaktose-Oxidase eingesetzt. Die vorteilhaften Ergebnisse sind in Fig. 3 veranschaulicht.

In einer Ausführungsform ist die Peroxidase ausgewählt aus einer Peroxidase der Gruppe bestehend aus der Enzymklasse EC 1.11.1.7, insbesondere aus der Peroxidase aus Meerrettich (*Armoracia rusticana*)*.* Die Meerrettich-Peroxidase kann entweder kommerziell bezogen werden (SIGMA-ALDRICH, z.B. Produktnummer: P8250), oder rekombinant hergestellt werden. Die Hinterlegungsnummern bei der GenBank lauten X57564.1 für die mRNA der Nukleinsäuresequenz, bzw. CAA40796.1 für die Polypeptidsequenz für das Enzym aus *Armoracia rusticana.*

In einer weiteren Ausführungsform der vorliegenden Erfindung ist die Katalase ausgewählt aus einer Katalase der Gruppe bestehend aus der Enzymklasse EC 1.11.1.6, insbesondere aus der Katalase aus Rind (*Bos taurus*)*.*

In einer Ausführungsform ist die Katalase Rinderleberkatalase der Enzymklasse EC 1.11.1.6 (Quelle etwa SIGMA ALDRICH, Produktnummer: C30), welche geeignet zur direkten enzymatischen Umsetzung ist. Zur rekombinanten Expression ist die Nukleinsäuresequenz einer beispielhaften Rinderleberkatalase öffentlich zugänglich als NCBI Bezugssequenz NM_001035386.2 (mRNA), die entsprechende translatierte Polypeptidsequenz ist öffentlich zugänglich als NCBI Bezugssequenz NP_001030463.1.

Zur fermentativen Erzeugung von Enzymen gemäß der vorliegenden Offenbarung können auch Galaktose-Oxidase und/oder Peroxidase und/oder Katalase Sequenzen mit mindestens 80%, 81%, 82%, 83%, 84%, 85%, 86%, 87%, 88%, 89%, 90%, 91%, 92%, 93%, 94%, 95%, 96%, 97%, 98% oder 99% Sequenzhomologie zu den oben aufgeführten Datenbank-Sequenzen verwendet werden, wobei die Nukleinsäure- oder die Polypeptidsequenzen Codon-optimiert sein können, oder gezielte Punktmutationen enthalten können, solange das translatierte Polypeptid nach wie vor die Funktion der Wildtyp-Bezugssequenz erfüllt. Derartige Modifikationen zur Anpassung der Nukleinsäure- und damit der Polypeptidsequenz eines Enzyms sind dem Fachmann auf dem Gebiet wohlbekannt und dienen unter anderem etwa der Anpassung der Sequenz an den Codon-Usage des zur Expression gewählten rekombinanten Wirtsorganismus, der Optimierung der Enzymstabilität oder -funktion sowie der Erhöhung der Ausbeute oder der Erniedrigung der Zytotoxizität des Enzymprodukts für den gewählten Wirtsorganismus.

Ferner können die Nukleinsäure- oder Polypetidsequenzen Markierungssequenzen ("Tags") tragen, welche die Aufreinigung oder Detektion der translatierten Polypeptidsequenzen, welche als Fusionsmolekül mit dem Tag translatiert werden, gestatten. Solche Tags und deren Sequenzen sowie Verfahren zur Modifikation einer Zielsequenz sind dem Fachmann auf dem Gebiet wohlbekannt und umfassen Sequenzen wie ein Streptavidin-Tag, poly-Histidin-Tag, Glutathion-S-Transferase-Tag, Maltose binding protein-Tag, Flash-Tag, Tags, welche die Sekretion des rekombinanten Polypeptids in den Kulturüberstand mediieren, und dergleichen.

In einer Ausführungsform der vorliegenden Erfindung ist die Konzentration an Galaktose-Oxidase in der Mischung zu Beginn der Inkubation in Schritt (c) im Bereich von 0,005 bis 6,25 g / L, vorzugsweise im Bereich von 0,005 bis 3,2 g / L, noch bevorzugter im Bereich von 0,005 bis 0,5 g / L und ganz besonders bevorzugt im Bereich von 0,005 bis 0,05 g / L. Es ist dem Fachmann auf dem Gebiet bekannt, dass die Konzentration der eingesetzten Galaktose-Oxidase abhängig von dem spezifisch eingesetzten Enzym sowie dessen spezifischer Enzymaktivität ist. Die Enzymaktivität kann in zwei möglichen Einheiten angegeben werden: (1) in Units (1 Enzymeinheit) = 1µmol Substrat min⁻¹, oder als SI-Einheit in Katal (kat) = 1 mol Substrat s⁻¹. Dem Fachmann auf dem Gebiet ist die näherungsweise Berechnung der Geschwindigkeit einer enzymatischen Reaktion und damit auch der Enzymaktivität durch die Michaelis-Menten-Gleichung bekannt, sodass in einem geeigneten Assay die Enzymaktivität für ein Enzym von Interesse leicht bestimmt werden kann.

In einer anderen Ausführungsform der vorliegenden Erfindung ist die Konzentration an Peroxidase in der Mischung zu Beginn der Inkubation in Schritt (c) nach dem ersten Aspekt im Bereich von 0 bis 0,5 g / L , vorzugsweise im Bereich von 0 bis 0,3 g / L, und noch bevorzugter im Bereich von 0 bis 0, 15 g / L.

In einer weiteren Ausführungsform der vorliegenden Erfindung ist die Konzentration an Katalase in der Mischung zu Beginn der Inkubation in Schritt (c) nach dem ersten Aspekt im Bereich von 0 bis 0,4 g / L, vorzugsweise im Bereich von 0 bis 0,2 g / L, und noch bevorzugter im Bereich von 0 bis 0,1 g / L.

In noch einer weiteren Ausführungsform der vorliegenden Erfindung ist die Konzentration an L-Fucitol in der Mischung zu Beginn der Inkubation in Schritt (c) nach dem ersten Aspekt im Bereich von 1 bis 500 g / L, vorzugsweise im Bereich von 5 bis 250 g / L, besonders bevorzugt im Bereich von 20 bis 125 g / L.

Der rekombinante Mikroorganismus oder Pilz kann aus der Gruppe bestehend aus *Escherichia coli* spp., bevorzugt *E. coli* BL21, *E. coli* MG1655 oder *E. coli* W3110 und deren Abkömmlinge, *Bacillus* spp., bevorzugt *Bacillus licheniformis, Bacillus subitilis* oder *Bacillus amyloliquefaciens,* und deren Abkömmlinge, Saccharomycetes, vorzugsweise der Ordnung der Saccharomycetales, weiter bevorzugt der Familie der Saccharomycetaceae, weiter bevorzugt der Gattung der *Komagataella*, bevorzugt *Hansenula* bzw. *Pichia* spp., besonders bevorzugt *P*. *pastoris* und deren Abkömmlinge, *Kluyveromyces* spp, bevorzugt *K*. *lactis,* und deren Abkömmlinge, *Aspergillus* spp., bevorzugt *A.* oryzae, *A. nidulans,* oder *A. niger,* und deren Abkömmlinge, oder Trichoderma spp., bevorzugt *T. reesei* oder *T. harzianum,* und deren Abkömmlinge, oder ein Pilz der Abteilung Ascomycota ist, bevorzugt *Myceliopthora thermophila,* oder ein Abkömmling davon ausgewählt werden. Gemäß einem Aspekt der vorliegenden Offenbarung wird ein rekombinanter Mikroorganismus oder Pilz in einem Verfahren zur Herstellung von L-Fucose aus L-Fucitol eingesetzt oder verwendet, wobei der Mikroorganismus oder Pilz ein Transgen, das für eine Galaktose-Oxidase aus einem Pilz der Gattung *Fusarium* kodiert, enthält, und weiter ein Transgen , das für eine Galaktose- Oxidase aus einem Pilz der Gattung Fusarium kodiert, enthält, wobei der rekombinante Mikroorganismus oder Pilz ausgewählt ist aus der Gruppe bestehend aus *Escherichia coli* spp., bevorzugt *E. coli* BL21, *E. coli* MG1655 oder *E*. *coli* W3110 und deren Abkömmlinge, *Bacillus* spp., bevorzugt *Bacillus licheniformis, Bacillus subitilis* oder *Bacillus amyloliquefaciens,* und deren Abkömmlinge, Saccharomycetes, vorzugsweise der Ordnung der Saccharomycetales, weiter bevorzugt der Familie der Saccharomycetaceae, weiter bevorzugt der Gattung der *Komagataella*, bevorzugt *Hansenula* bzw. *Pichia* spp., besonders bevorzugt *P. pastoris* und deren Abkömmlinge, *Kluyveromyces* spp, bevorzugt *K. lactis,* und deren Abkömmlinge, *Aspergillus* spp., bevorzugt *A.* oryzae, *A. nidulans,* oder *A. niger,* und deren Abkömmlinge, oder Trichoderma spp., bevorzugt *T. reesei* oder *T. harzianum,* und deren Abkömmlinge, oder ein Pilz der Abteilung Ascomycota ist, bevorzugt *Myceliopthora thermophila,* oder ein Abkömmling davon, und wobei der rekombinante Mikroorganismus oder Pilz das oder die Transgen(e) in funktionaler Form exprimieren kann.

Dem Fachmann auf dem Gebiet ist wohlbekannt, dass der Begriff "geeignete (Reaktions-) Bedingungen" im Kontext von Enzymen impliziert, dass die notwendigen Substrate, Pufferbedingungen, speziell Salzkonzentrationen, ggf. Kofaktoren, umfassend unter anderem prosthetische Gruppen, Coenzyme und Metall-Ionen, und pH- sowie Temperatur-Bedingungen bereitgestellt werden müssen, welche für die Aktivität des entsprechenden Enzyms unerlässlich oder förderlich sind. Im Falle der Verwendung einer Galaktose-Oxidase - einem Sauerstoff-abhängigen Enzym - umfasst dies zum Beispiel Aeration durch Begasen oder Belüften eines Kultur- oder Reaktionsansatzes. Da alle Enzyme, die in in dem Verfahren der vorliegenden Erfindung eingesetzt oder verwendet werden sowie die von ihnen katalysierte Umsetzung gut charakterisiert sind, ist die Bestimmung geeigneter Reaktionsbedingungen daher innerhalb der Fähigkeiten des Fachmanns auf diesem Gebiet.

In einer Ausführungsform ist neben dem Transgen, das für eine Galaktose-Oxidase kodiert, ein weiteres Transgen, das für eine Katalase kodiert, in dem rekombinanten Mikroorganismus oder Pilz enthalten.

In einer anderen Ausführungsform ist neben dem Transgen, das für eine Galaktose-Oxidase kodiert, ein weiteres Transgen, das für eine Peroxidase kodiert, in dem rekombinanten Mikroorganismus oder Pilz enthalten.

In noch einer anderen Ausführungsform ist neben dem Transgen, das für eine Galaktose-Oxidase kodiert, sowohl ein weiteres Transgen, das für eine Peroxidase kodiert, als auch ein Transgen, das für eine Katalase kodiert, in dem rekombinanten Mikroorganismus oder Pilz enthalten.

Die Bereitstellung des jeweiligen Transgens, dessen Einbringung in den rekombinanten Mikroorganismus oder Pilz sowie die Auswahl geeigneter Kultur- und Reaktionsbedingungen, die die Tranksription und Translation des jeweiligen Transgens gestatten, sind dem Fachmann auf dem Gebiert wohlbekannt.

Der rekombinante Mikroorganismus oder Pilz kann durch Zugabe von L-Fucitol zum Kulturmedium zur unmittelbaren fermentativen Herstellung von L-Fucose aus L-Fucitol verwendet werden, d.h. der rekombinante Mikroorganismus oder Pilz wird unmittelbar zur Durchführung des oben beschriebenen Verfahrens eingesetzt. Diese Ausführungsform erlaubt die direkte Herstellung von L-Fucose, optional gefolgt von weiteren Reinigungsschritten, im Fermentationsansatz.

Die Transgene für die Galaktose-Oxidase sowie die Peroxidase und/oder die Katalase können mit einem Tag versehen werden, welches dies Sekretion der translatierten Polypeptide in den Kulturüberstand erlaubt. Dies erlaubt entweder die leichte Isolierung der Enzyme aus dem Kulturüberstand oder die direkte Durchführung der Umsetzung von L-Fucitol zu L-Fucitose nach Zugabe von L-Fucitol und die Anpassung der Reaktionsbedingungen, so dass die sekretierten Enzyme ihre enzymatische Funktion erfüllen können.

In einer anderen Ausführungsform werden die Transgene die für eine Galaktose-Oxidase, eine Peroxidase und/oder eine Katalase kodieren, nicht in einem, sondern in unterschiedlichen rekombinanten Mikroorganismen oder Pilzen bereitgestellt und unter geeigneten Kulturbedingungen exprimiert und optional gereinigt. Hierauf kann mit den so erhaltenen Enzymen *in vitro* oder *in vivo* nach Zugabe von L-Fucitol das hierin beschriebene Verfahren durchgeführt werden, so dass durch Bereitstellung von geeigneten Reaktionsbedingungen L-Fucose aus L-Fucitol gewonnen werden kann.

Die Erfindung stellt die Verwendung eines rekombinanten Mikroorganismus oder Pilzes bereit, wobei der Mikroorganismus oder Pilz ein Transgen, das für eine Galaktose-Oxidase aus einem Pilz der Gattung Fusarium kodiert, wie oben definiert, enthält, zur rekombinanten Expression einer Galaktose-Oxidase, in einem Verfahren wie oben beschrieben, wobei der rekombinante Mikroorganismus oder Pilz ausgewählt ist aus der Gruppe bestehend aus *Escherichia coli* spp., bevorzugt *E*. *coli* BL21, *E*. *coli* MG1655 oder *E*. *coli* W3110 und deren Abkömmlinge, *Bacillus* spp., bevorzugt *Bacillus licheniformis, Bacillus subitilis* oder *Bacillus amyloliquefaciens,* und deren Abkömmlinge, Saccharomycetes, vorzugsweise der Ordnung der Saccharomycetales, weiter bevorzugt der Familie der Saccharomycetaceae, weiter bevorzugt der Gattung der *Komagataella*, bevorzugt *Hansenula* bzw. *Pichia* spp., besonders bevorzugt *P*. *pastoris* und deren Abkömmlinge, *Kluyveromyces* spp, bevorzugt *K*. *lactis,* und deren Abkömmlinge, *Aspergillus* spp., bevorzugt *A.* oryzae, *A. nidulans,* oder *A. niger,* und deren Abkömmlinge, oder Trichoderma spp., bevorzugt *T. reesei* oder *T. harzianum,* und deren Abkömmlinge, oder ein Pilz der Abteilung Ascomycota ist, bevorzugt *Myceliopthora thermophila,* oder ein Abkömmling davon, und wobei der rekombinante Mikroorganismus oder Pilz das oder die Transgen(e) in funktionaler Form exprimieren kann.

Die Erfindung stellt die Verwendung einer Galaktose-Oxidase der Enzymklasse EC 1.1.3.9 sowie einer Peroxidase und einer Katalase, bereit, wobei die Galaktose-Oxidase sowie die Peroxidase und die Katalase durch ein Verfahren umfassend folgende Schritte erhalten werden:
(a) Bereitstellen eines rekombinanten Mikroorganismus oder Pilzes zur rekombinanten Expression von einer Galaktose-Oxidase und einer Peroxidase und einer Katalase, wobei der Mikroorganismus ein Transgen, das für eine Galaktose-Oxidase aus einem Pilz der Gattung Fusarium kodiert, enthält, und mindestens ein weiteres Transgen, das für eine Peroxidase und Katalase kodiert, enthält, und wobei der rekombinante Mikroorganismus oder Pilz ausgewählt ist aus der Gruppe bestehend aus *Escherichia coli* spp., bevorzugt *E. coli* BL21, *E. coli* MG1655 oder *E. coli* W3110 und deren Abkömmlinge, *Bacillus* spp., bevorzugt *Bacillus licheniformis, Bacillus subitilis* oder *Bacillus amyloliquefaciens,* und deren Abkömmlinge, Saccharomycetes, vorzugsweise der Ordnung der Saccharomycetales, weiter bevorzugt der Familie der Saccharomycetaceae, weiter bevorzugt der Gattung der *Komagataella*, bevorzugt *Hansenula* bzw. *Pichia* spp., besonders bevorzugt *P. pastoris* und deren Abkömmlinge, *Kluyveromyces* spp, bevorzugt *K. lactis,* und deren Abkömmlinge, *Aspergillus* spp., bevorzugt *A.* oryzae, *A. nidulans,* oder *A. niger,* und deren Abkömmlinge, oder Trichoderma spp., bevorzugt *T. reesei* oder *T. harzianum,* und deren Abkömmlinge, oder ein Pilz der Abteilung Ascomycota ist, bevorzugt *Myceliopthora thermophila,* oder ein Abkömmling davon, oder
(b1) Bereitstellen eines ersten rekombinanten Mikroorganismus oder Pilzes zur rekombinanten Expression einer Galaktose-Oxidase, wobei der Mikroorganismus oder Pilz ein Transgen, das für eine Galaktose-Oxidase aus einem Pilz der Gattung Fusarium kodiert, enthält; und
(b2) Bereitstellen eines zweiten rekombinanten Mikroorganismus oder Pilzes zur rekombinanten Expression einer Peroxidase, wobei der Mikroorganismus oder Pilz ein Transgen, das für eine Peroxidase, kodiert, enthält; und
(b3) Bereitstellen eines dritten rekombinanten Mikroorganismus oder Pilzes zur rekombinanten Expression einer Katalase, wobei der Mikroorganismus oder Pilz ein Transgen, das für eine Katalase, kodiert, enthält;
(c) Kultivieren des rekombinanten Mikroorganismus unter geeigneten Bedingungen, die die Synthese der Galaktose-Oxidase und der Peroxidase und/oder der Katalase erlauben,
(d) optional: Isolieren der synthetisierten Galaktose-Oxidase und der Peroxidase und/oder der Katalase, zur biokatalytischen Umsetzung von L-Fucitol zu L-Fucose, in einem Verfahren nach dem ersten Aspekt der vorliegenden Erfindung.

Für die Peroxidase und die Katalase gilt daher jeweils das oben Gesagte entsprechend.

Dieser Aspekt der vorliegenden Erfindung ist speziell nützlich, um alle erwünschten Einzelenzyme zur Durchführung der biokatalytischen Umsetzung von L-Fucitol zu L-Fucose individuell in einem oder in mehreren rekombinanten Organismus/en bereitzustellen, herzustellen und optional zu isolieren, um die Umsetzung von L-Fucitol zu L-Fucose im Anschluss gezielt enzymatisch *in vitro* durchzuführen.

### Beispiele

Die Erfindung wird nun näher durch die folgenden Beispiele veranschaulicht.

### Beispiel 1: Herstellung von L-Fucose durch biokatalytische Oxidation von L-Fucitol mit Galaktose-Oxidase in Anwesenheit von Peroxidase und Katalase

Zu einer Rundboden Dreihalsflasche (50 mL) wurde eine Lösung von L-Fucitol (6,0 mL wässrige Lösung enthaltend 600 mg L-Fucitol, CAS 13074-06-1, Santa Cruz Biotechnology) hinzugefügt, gefolgt von Zugabe von 1,2 mL K₂HPO₄/KH₂PO₄ (1000 mM, pH=7,0) sowie von 0,095 mL Katalase (aus Rinderleber, SIGMA, 21.300 U/mg, 34mg/mL), Zugabe von 0,120 mL Peroxidase (aus Meerrettich, 173 U/mg fest, SIGMA) und von 2,218 mL Galaktose-Oxidase (38,4 mg/mL, 2.708 U/mL). Die resultierende Lösung wurde bei Raumtemperatur mit O₂ durchspült, bis das gesamte L-Fucitol zu L-Fucose umgewandelt worden war. Die Reaktion wurde mittels HLPC überwacht. Das finale Produkt wurde isoliert und mittels 1H und 13C-NMR analysiert. Die Ergebnisse sind in Tabelle 1 zusammengefasst.

**[Tabelle 1]**

| **Reaktionszeit [h]** | **Umwandlung [%]** |
|---|---|
| 0 | - |
| 3,5 | 54,0 |
| 24 | 95,8 |
| 29 | 96,0 |

### Beispiel 2: Einfluss von Peroxidase und Katalase auf die biokatalvtische Oxidation von L-Fucitol

Die folgenden Experimente wurden im 1,0 mL Maßstab durchgeführt: Galaktose-Oxidase (GO): Zu einer 0,250 mL L-Fucitol-Lösung (100 mg/mL) wurden 0,635 mL Wasser, gefolgt von 0,050 mL K₂HPO₄ / KH₂PO₄ Pufferlösung, pH 7,0, 0,065 mL Galaktose-Oxidase (2.705 U/mL in 100 mM K₃PO₄ Puffer, pH 6,0) hinzugefügt; GO + Katalase: Zu einer 0,250 mL L-Fucitol-Lösung (100 mg/mL) wurden 0,631 mL Wasser, gefolgt von 0,050 mL K₂HPO₄ / KH₂PO₄ Puffer, pH 7,0, 0,004 mL Katalase in Wasser (724.200 U/mL), 0,065 mL Galaktose-Oxidase (2.705 U/mL in 100mM K₃PO₄ Puffer, pH 6,0, hinzugefügt. GO + Peroxidase: Zu einer 0,250 mL L-Fucitol-Lösung (100 mg/mL) wurden 0,630 mL Wasser gefolgt von 0,050 mL K₂HPO₄ / KH₂PO₄ Puffer, pH 7,0, 0,005 mL Peroxidase in Wasser (7.500 U/mL), 0,065 mL Galaktose- Oxidase (2.705 U/mL in 100mM K₃PO₄ Puffer, pH 6,0), hinzugefügt; GO + Peroxidase + Katalase: Zu einer 0,250 mL L-Fucitol-Lösung (100 mg/mL) wurden 0,626 mL Wasser gefolgt von 0,050 mL K₂HPO₄ / KH₂PO₄ Puffer, pH 7,0, 0,005 mL Peroxidase in Wasser (7.500 U/mL), 0,004 mL Katalase in Wasser (724.200 U/mL), 0,065 mL Galaktose- Oxidase (2.705 U/mL in 100mM K₃PO₄ Puffer, pH 6,0), hinzugefügt. Alle Reaktionen wurden mit Sauerstoff durchspült und für 60 min gerührt. Der Ablauf der Reaktion wurde mittels HPLC überwacht. Die Ergebnisse der unterschiedlichen Reaktionen sind in Fig. 3 zusammengefasst.

### Beispiel 3: Einfluss der L-Fucitol-Konzentration auf die biokatalvtische Oxidation von L-Fucitol

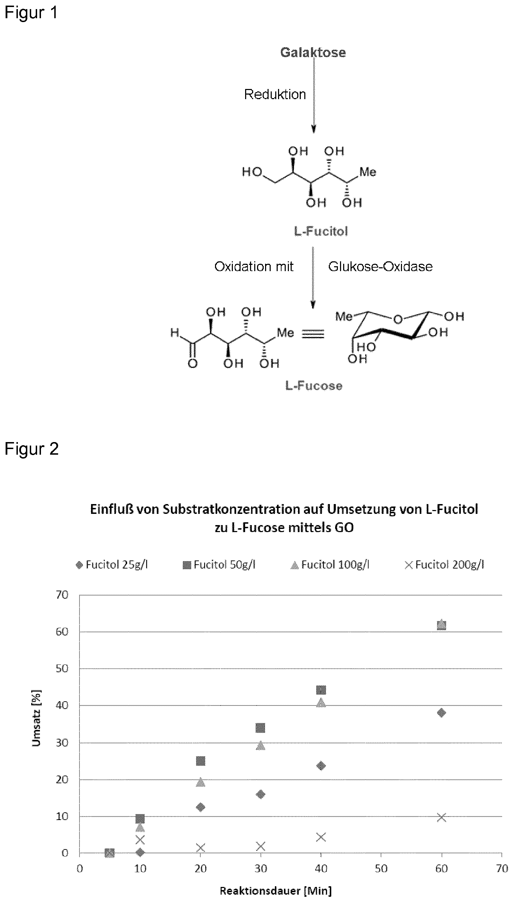

Die folgenden Experimente wurden im 1,0 mL Maßstab durchgeführt: **25 g/L L-Fucitol:** Zu einer 0,100 mL L-Fucitol-Lösung (100mg/mL) wurden 0,231 mL Wasser, gefolgt von 0,040 ml K₂HPO₄ / KH₂PO₄ Pufferlösung, pH 7,0, 0,005 mL Peroxidase in Wasser (7.500 U/mL), 0,004 ml Katalase in Wasser (724.200 U/mL), 0,065 mL Galaktose-Oxidase (2.705 U/mL in 100mM in K3PO4 Puffer, pH 6,0), hinzugefügt. **50 g/L L-Fucitol:** 0,020 g L-Fucitol wurden zu 0,281 mL Wasser, gefolgt von 0,040 ml K2HPO4 / KH2PO4 Pufferlösung, pH 7,0, 0,004 mL Peroxidase in Wasser (7.500 U/mL), 0,0032 mL Katalase in Wasser (724.200 U/mL), 0,052 mL Galaktose-Oxidase (2.705 U/mL in 100mM in K3PO4 Puffer, pH 6,0), hinzugefügt. **100 g/L L-Fucitol:** 0,040 g L-Fucitol wurde zu 0,202 mL Wasser, gefolgt von 0,040 ml K2HPO4 / KH2PO4 Pufferlösung pH 7,0, 0,0080 mL Peroxidase in Wasser (7.500 U/mL), 0,0064 ml Katalase in Wasser (724.200 U/mL), 0,104 mL Galaktose-Oxidase (2.705 U/mL in 100mM in K3PO4 Puffer, pH 6,0), hinzugefügt. **200g/L L-Fucitol:** 0,080 g L-Fucitol wurde zu 0,044 mL Wasser, gefolgt von 0,040 ml K2HPO4 / KH2PO4 Pufferlösung, pH 7,0, 0,0160 mL Peroxidase in Wasser (7.500 U/mL), 0,0127 mL Katalase in Wasser (724.200 U/mL), 0,207 mL Galaktose-Oxidase (2.705 U/mL in 100mM K3PO4 Puffer, pH 6,0). Alle Reaktionen wurden mit Sauerstoff durchspült und für 60 min gerührt. Der Ablauf der Reaktion wurde mittels HPLC überwacht. Die Ergebnisse sind in Fig. 2 zusammengefasst.

Fig.4 zeigt ein weiteres Kontrollexperiment, bei welchem zusätzlich der Einfluss von L-Fucose auf die Umsetzung von L-Fucitol untersucht wurde.

### Beispiel 4: Fermentative Herstellung und Gewinnung von Galaktose-Oxidase

*Pichia pastoris* wurde mit einem geeigneten Vektor, in welchen das Wildtyp-Galaktose-Oxidase Gen von *Fusarium* spp. kloniert wurde, transformiert. Als Vektor erwies sich jeder verfügbare *Pichia* kompatible Vektor als nützlich. Zusätzlich wurden unterschiedliche Tags verwendet, entweder, um die Aufreinigung des erhaltenen Enzyms zu unterstützen und/oder dessen Sekretion in den Kulturüberstand zu bewirken. Als Promotor wurde ein Methanol-induzierbarer Promotor gewählt. Dieser zeigte sich im Fermenter-Maßstab für *Pichia* als Wirtsorganismus als geeignet, die Expression großer Mengen an Galaktose-Oxidase zu induzieren. Die erhaltene Galaktose-Oxidase wurde gegebenenfalls isoliert bzw. aus dem Kulturüberstand gewonnen und optional gereinigt. Die gewonnene Galaktose-Oxidase hatte eine Aktivität von etwa 3.000 U/g. Im Falle der Verwendung eines filamentösen Pilzes als rekombinanten Pilz wurde ebenfalls die Sekretion der Galaktose-Oxidase direkt in den Kulturüberstand bewirkt.

Es wurden weitere Beispiele mit weiteren Expressionsstämmen, umfassend *E. coli,* und verschiedenen wohlbekannten Pilzstämmen unternommen und die verwendeten Vektoren, Kontrollelemente, Medien und Kulturbedingungen wurden entsprechend angepasst. Diese Modifikationen sind dem Fachmann auf dem Gebiet wohlbekannt. All diese Expressionen ergaben Galaktose-Oxidase in ausreichender Menge, Reinheit und mit ausreichender Aktivität, um die biokatalytische Herstellung von L-Fucose zu katalysieren. Als besonders bevorzugte Stämme haben sich Hefe- und Pilzstämme erwiesen.

## Patentansprüche

1. Verfahren zur Herstellung von L-Fucose, umfassend folgende Schritte:
(a) Bereitstellen von L-Fucitol,
(b) Bereitstellen einer Galaktose-Oxidase der Enzymklasse EC 1.1.3.9, einer Peroxidase und einer Katalase,
(c) Oxidation von L-Fucitol unter Verwendung der Galaktose-Oxidase durch Inkubieren der erhaltenen Mischung unter Bedingungen, die die biokatalytische Oxidation von L-Fucitol zu L-Fucose erlauben,
(d) optional: Isolieren der synthetisierten L-Fucose.

2. Verfahren nach Anspruch 1, wobei Schritt (b) folgende Teilschritte umfasst oder daraus besteht:
(b1) Bereitstellen eines rekombinanten Mikroorganismus oder Pilzes zur rekombinanten Expression von einer Galaktose-Oxidase der Enzymklasse EC 1.1.3.9, wobei der Mikroorganismus oder Pilz ein Transgen, das für eine Galaktose-Oxidase aus einem Pilz der Ordnung der *Hypocreales* kodiert, enthält,
(b2) Kultivieren des rekombinanten Mikroorganismus unter Bedingungen, die die Synthese von einer Galaktose-Oxidase erlauben,
(b3) optional: Isolieren der synthetisierten Galaktose-Oxidase.

3. Verfahren nach Anspruch 2, wobei der rekombinante Mikroorganismus oder Pilz ausgewählt ist aus der Gruppe bestehend aus *Escherichia coli* spp. und deren Abkömmlinge, *Bacillus* spp. und deren Abkömmlinge, Saccharomycetes und deren Abkömmlinge, *Kluyveromyces* spp. und deren Abkömmlinge, *Aspergillus* spp. und deren Abkömmlinge, Trichoderma spp. und deren Abkömmlinge, oder einem Pilz der Abteilung Ascomycota oder einem Abkömmling davon, wobei der rekombinante Mikroorganismus oder Pilz das oder die Transgen(e) in funktionaler Form exprimieren kann.

4. Verfahren nach Anspruch 2 oder 3, wobei der rekombinante Mikroorganismus ausgewählt ist aus der Gruppe bestehend aus der Gattung *Komagataella*, bevorzugt wobei der rekombinante Mikroorganismus *Hansenula* bzw. *Pichia spp.,* besonders bevorzugt *Pichia pastoris,* ist.

5. Verfahren nach einem der vorangehenden Ansprüche, wobei die Peroxidase ausgewählt ist aus einer Peroxidase der Gruppe bestehend aus der Enzymklasse EC 1.11.1.7, insbesondere aus der Peroxidase aus Meerrettich (*Armoracia rusticana*)*.*

6. Verfahren nach einem der vorangehenden Ansprüche, wobei die Katalase ausgewählt ist aus einer Katalase der Gruppe bestehend aus der Enzymklasse EC 1.11.1.6, insbesondere aus der Katalase aus Rind *(Bos taurus).*

7. Verfahren nach einem der vorangehenden Ansprüche, wobei die Konzentration an L-Fucitol in der Mischung zu Beginn der Inkubation in Schritt (c) im Bereich von 1 bis 500 g / L liegt, vorzugsweise im Bereich von 5 bis 250 g / L, besonders bevorzugt im Bereich von 20 bis 125 g / L.

8. Verfahren nach einem der vorangehenden Ansprüche, wobei die Konzentration an Galaktose-Oxidase in der Mischung zu Beginn der Inkubation in Schritt (c) im Bereich von 0,005 bis 6,25 g / L liegt, vorzugsweise im Bereich von 0,005 bis 3,2 g / L, noch bevorzugter im Bereich von 0,005 bis 0,5 g / L und ganz besonders bevorzugt im Bereich von 0,005 bis 0,05 g / L liegt.

9. Verfahren nach einem der vorangehenden Ansprüche, wobei die Konzentration an Peroxidase in der Mischung zu Beginn der Inkubation in Schritt (c) im Bereich von 0 bis 0,5 g / L, vorzugsweise im Bereich von 0 bis 0,3 g / L, und noch bevorzugter im Bereich von 0 bis 0, 15 g / L, liegt.

10. Verfahren nach einem der vorangehenden Ansprüche, wobei die Konzentration an Katalase in der Mischung zu Beginn der Inkubation in Schritt (c) im Bereich von 0 bis 0,4 g / L, vorzugsweise im Bereich von 0 bis 0,2 g / L, und noch bevorzugter im Bereich von 0 bis 0,1 g / L, liegt.

11. Verfahren nach Anspruch 2, wobei der Mikroorganismus oder Pilz ein Transgen enthält, das für eine Galaktose-Oxidase aus einem Pilz der Gattung *Fusarium,* insbesondere der Species *Fusarium oxysporum,* kodiert.

12. Verwendung eines rekombinanten Mikroorganismus oder Pilzes wie definiert in einem der Ansprüche 3 oder 4, in einem Verfahren nach einem der Ansprüche 1 bis 11.

13. Verwendung einer Galaktose-Oxidase der Enzymklasse EC 1.1.3.9 sowie einer Peroxidase und einer Katalase
zur biokatalytischen Umsetzung von L-Fucitol zu L-Fucose, wobei die Galaktose-Oxidase sowie die Peroxidase und die Katalase erhalten werden durch ein Verfahren umfassend folgende Schritte:
(a) Bereitstellen eines rekombinanten Mikroorganismus oder Pilzes zur rekombinanten Expression von einer Galaktose-Oxidase und einer Peroxidase und einer Katalase, wobei der Mikroorganismus ein Transgen, das für eine Galaktose-Oxidase aus einem Pilz der Gattung Fusarium kodiert, enthält, und mindestens ein weiteres Transgen, das für eine Peroxidase und Katalase kodiert, enthält, und wobei der rekombinante Mikroorganismus oder Pilz wie in einem der Ansprüche 3 oder 4 definiert ist, oder
(b1) Bereitstellen eines ersten rekombinanten Mikroorganismus oder Pilzes zur rekombinanten Expression einer Galaktose-Oxidase, wobei der Mikroorganismus oder Pilz ein Transgen, das für eine Galaktose-Oxidase aus einem Pilz der Gattung Fusarium kodiert, enthält; und
(b2) Bereitstellen eines zweiten rekombinanten Mikroorganismus oder Pilzes zur rekombinanten Expression einer Peroxidase, wobei der Mikroorganismus oder Pilz ein Transgen, das für eine Peroxidase, wie in Anspruch 5 definiert, kodiert, enthält; und
(b3) Bereitstellen eines dritten rekombinanten Mikroorganismus oder Pilzes zur rekombinanten Expression einer Katalase, wobei der Mikroorganismus oder Pilz ein Transgen, das für eine Katalase, wie in Anspruch 6 definiert, kodiert, enthält;
(c) Kultivieren des/der rekombinanten Mikroorganismus/en unter geeigneten Bedingungen, die die Synthese der Galaktose-Oxidase und der Peroxidase und der Katalase erlauben,
(d) optional: Isolieren der synthetisierten Galaktose-Oxidase und der Peroxidase und der Katalase.

## Claims

1. A method for producing L-fucose, comprising the following steps:
(a) providing L-fucitol,
(b) providing a galactose oxidase of the enzyme class EC 1.1.3.9, a peroxidase and a catalase,
(c) oxidation of L-fucitol using the galactose oxidase by incubating the resulting mixture under conditions permitting the biocatalytic oxidation of L-fucitol to L-fucose,
(d) optionally: isolating the synthesized L-fucose.

2. The method according to claim 1, wherein step (b) comprises or consists of the following substeps:
(b1) providing a recombinant microorganism or fungus for the recombinant expression of a galactose oxidase of the enzyme class EC 1.1.3.9, wherein the microorganism or fungus comprises a transgene which encodes a galactose oxidase from a fungus of the order of the *Hypocreales,*
(b2) culturing the recombinant microorganism under conditions which permit the synthesis of a galactose oxidase,
(b3) optionally: isolating the synthesized galactose oxidase.

3. The method according to claim 2, wherein the recombinant microorganism or fungus is selected from the group consisting of *Escherichia coli* spp. and descendants thereof, *Bacillus* spp. and descendants thereof, Saccharomycetes and descendants thereof, *Kluyveromyces* spp. and descendants thereof, *Aspergillus* spp. and descendants thereof, Trichoderma spp. and descendants thereof, or a fungus of the phylum Ascomycota or a descendant thereof, wherein the recombinant microorganism or fungus can express the transgene(s) in functional form.

4. The method according to claim 2 or 3, wherein the recombinant microorganism is selected from the group consisting of the genus *Komagataella,* preferably wherein the recombinant microorganism is *Hansenula* or *Pichia* spp., particularly preferably *Pichia pastoris.*

5. The method according to any of the preceding claims, wherein the peroxidase is selected from a peroxidase of the group consisting of the enzyme class EC 1.11.1.7, in particular from the peroxidase from horseradish (*Armoracia rusticana*).

6. The method according to any of the preceding claims, wherein the catalase is selected from a catalase of the group consisting of the enzyme class EC 1.11.1.6, in particular from the catalase from cattle (*Bos taurus*)*.*

7. The method according to any of the preceding claims, wherein the concentration of L-fucitol in the mixture at the start of the incubation in step (c) is in the range from 1 to 500 g/L, preferably in the range from 5 to 250 g/L, particularly preferably in the range from 20 to 125 g/L.

8. The method according to any of the preceding claims, wherein the concentration of galactose oxidase in the mixture at the start of the incubation in step (c) is in the range from 0.005 to 6.25 g/L, preferably in the range from 0.005 to 3.2 g/L, even more preferably in the range from 0.005 to 0.5 g/L and especially preferably in the range from 0.005 to 0.05 g/L.

9. The method according to any of the preceding claims, wherein the concentration of peroxidase in the mixture at the start of the incubation in step (c) is in the range from 0 to 0.5 g/L, preferably in the range from 0 to 0.3 g/L and even more preferably in the range from 0 to 0.15 g/L.

10. The method according to any of the preceding claims, wherein the concentration of catalase in the mixture at the start of the incubation in step (c) is in the range from 0 to 0.4 g/L, preferably in the range from 0 to 0.2 g/L and even more preferably in the range from 0 to 0.1 g/L.

11. The method according to claim 2, wherein the microorganism or fungus comprises a transgene which encodes a galactose oxidase from a fungus of the genus *Fusarium,* especially the species *Fusarium oxysporum.*

12. The use of a recombinant microorganism or fungus as defined in either of claims 3 and 4, in a method according to any of claims 1 to 11.

13. The use of a galactose oxidase of the enzyme class EC 1.1.3.9 and also a peroxidase and a catalase for the biocatalytic conversion of L-fucitol to L-fucose, wherein the galactose oxidase and the peroxidase and the catalase are obtained by a method comprising the following steps:
(a) providing a recombinant microorganism or fungus for the recombinant expression of a galactose oxidase and a peroxidase and a catalase, wherein the microorganism comprises a transgene which encodes a galactose oxidase from a fungus of the genus Fusarium, and at least one further transgene which encodes a peroxidase and catalase, and wherein the recombinant microorganism or fungus is defined as in either of claims 3 and 4, or
(b1) providing a first recombinant microorganism or fungus for the recombinant expression of a galactose oxidase, wherein the microorganism or fungus comprises a transgene encoding a galactose oxidase from a fungus of the genus Fusarium; and
(b2) providing a second recombinant microorganism or fungus for the recombinant expression of a peroxidase, wherein the microorganism or fungus comprises a transgene encoding a peroxidase as defined in claim 5; and
(b3) providing a third recombinant microorganism or fungus for the recombinant expression of a catalase, wherein the microorganism or fungus comprises a transgene encoding a catalase as defined in claim 6;
(c) culturing the recombinant microorganism(s) under suitable conditions which permit the synthesis of the galactose oxidase and the peroxidase and the catalase,
(d) optionally: isolating the synthesized galactose oxidase and the peroxidase and the catalase.

## Revendications

1. Procédé de préparation de L-fucose, comprenant les étapes suivantes :
(a) mise à disposition de L-fucitol,
(b) mise à disposition d'une galactose oxydase de la classe d'enzymes EC 1.1.3.9, d'une peroxydase et d'une catalase,
(c) oxydation de L-fucitol en utilisant la galactose oxydase par une incubation du mélange obtenu dans des conditions qui permettent l'oxydation biocatalytique de L-fucitol en L-fucose,
(d) éventuellement : isolement du L-fucose synthétisé.

2. Procédé selon la revendication 1, l'étape (b) comprenant les étapes partielles suivantes ou en étant constituée :
(b1) mise à disposition d'un micro-organisme ou champignon recombinant pour l'expression recombinante d'une galactose oxydase de la classe d'enzymes EC 1.1.3.9, le micro-organisme ou le champignon contenant un transgène qui code pour une galactose oxydase provenant d'un champignon d'ordre des *Hypocreales,*
(b2) culture du micro-organisme recombinant dans des conditions qui permettent la synthèse d'une galactose oxydase,
(b3) éventuellement : isolement de la galactose oxydase synthétisée.

3. Procédé selon la revendication 2, le micro-organisme ou champignon recombinant étant choisi dans le groupe constitué par *Escherichia coli* spp. et leurs dérivés, *Bacillus* spp. et leurs dérivés, Saccharomycetes et leurs dérivés, *Kluyveromyces* spp. et leurs dérivés, *Aspergillus* spp. et leurs dérivés, *Trichoderma* spp. et leurs dérivés, ou un champignon de la division Ascomycota ou un dérivé correspondant, le micro-organisme ou champignon recombinant pouvant exprimer le ou les transgènes sous forme fonctionnelle.

4. Procédé selon la revendication 2 ou 3, le micro-organisme recombinant étant choisi dans le groupe constitué par le genre *Komagataella*, préférablement le micro-organisme recombinant étant *Hansenula* ou *Pichia spp.,* particulièrement préférablement *Pichia pastoris.*

5. Procédé selon l'une quelconque des revendications précédentes, la peroxydase étant choisie parmi une peroxydase du groupe constitué par la classe d'enzymes EC 1.11.1.7, en particulier parmi la peroxydase de raifort (*Armoracia rusticana).*

6. Procédé selon l'une quelconque des revendications précédentes, la catalase étant choisie parmi une catalase du groupe constitué par la classe d'enzymes EC 1.11.1.6, en particulier parmi la catalase de bœuf (*Bos taurus*).

7. Procédé selon l'une quelconque des revendications précédentes, la concentration de L-fucitol dans le mélange au début de l'incubation dans l'étape (c) se situant dans la plage de 1 à 500 g/L, de préférence dans la plage de 5 à 250 g/L, particulièrement préférablement dans la plage de 20 à 125 g/L.

8. Procédé selon l'une quelconque des revendications précédentes, la concentration de galactose oxydase dans le mélange au début de l'incubation dans l'étape (c) se situant dans la plage de 0,005 à 6,25 g/L, de préférence dans la plage de 0,005 à 3,2 g/L, plus préférablement dans la plage de 0,005 à 0,5 g/L et tout particulièrement préférablement dans la plage de 0,005 à 0,05 g/L.

9. Procédé selon l'une quelconque des revendications précédentes, la concentration de peroxydase dans le mélange au début de l'incubation dans l'étape (c) se situant dans la plage de 0 à 0,5 g/L, de préférence dans la plage de 0 à 0,3 g/L et encore plus préférablement dans la plage de 0 à 0,15 g/L.

10. Procédé selon l'une quelconque des revendications précédentes, la concentration de catalase dans le mélange au début de l'incubation dans l'étape (c) se situant dans la plage de 0 à 0,4 g/L, de préférence dans la plage de 0 à 0,2 g/L et encore plus préférablement dans la plage de 0 à 0,1 g/L.

11. Procédé selon la revendication 2, le micro-organisme ou champignon contenant un transgène qui code pour une galactose oxydase provenant d'un champignon du genre *Fusarium,* en particulier de l'espèce *Fusarium oxysporum.*

12. Utilisation d'un micro-organisme ou champignon recombinant comme défini dans l'une quelconque des revendications 3 et 4, dans un procédé selon l'une quelconque des revendications 1 à 11.

13. Utilisation d'une galactose oxydase de la classe d'enzymes EC 1.1.3.9 ainsi que d'une peroxydase et d'une catalase pour la transformation biocatalytique de L-fucitol en L-fucose, la galactose oxydase ainsi que la peroxydase et la catalase étant obtenues par un procédé comprenant les étapes suivantes :
(a) mise à disposition d'un micro-organisme ou champignon recombinant pour l'expression recombinante d'une galactose oxydase et d'une peroxydase et d'une catalase, le micro-organisme contenant un transgène qui code pour une galactose oxydase provenant d'un champignon du genre Fusarium, et contenant au moins un transgène supplémentaire qui code pour une peroxydase et une catalase, et le micro-organisme ou champignon recombinant étant tel que défini dans l'une quelconque des revendications 3 et 4, ou
(b1) mise à disposition d'un premier micro-organisme ou champignon recombinant pour l'expression recombinante d'une galactose oxydase, le micro-organisme ou champignon contenant un transgène qui code pour une galactose oxydase provenant d'un champignon du genre Fusarium ; et
(b2) mise à disposition d'un deuxième micro-organisme ou champignon recombinant pour l'expression recombinante d'une peroxydase, le micro-organisme ou champignon contenant un transgène qui code pour une peroxydase, comme défini dans la revendication 5 ; et
(b3) mise à disposition d'un troisième micro-organisme ou champignon recombinant pour l'expression recombinante d'une catalase, le micro-organisme ou champignon contenant un transgène qui code pour une catalase, comme défini dans la revendication 6 ;
(c) culture du ou des micro-organismes recombinants dans des conditions appropriées qui permettent la synthèse de la galactose oxydase et de la peroxydase et de la catalase,
(d) éventuellement : isolement de la galactose oxydase synthétisée et de la peroxydase et de la catalase.
